# EUROPÄISCHE PATENTSCHRIFT

(11) **EP 3 774 749 B1**
(45) Veröffentlichungstag und Bekanntmachung des Hinweises auf die Patenterteilung: **20.11.2024**
(21) Anmeldenummer: 19715085.7
(22) Anmeldetag: 02.04.2019
(51) Int. Cl.: C07D 275/03

(54) **VERFAHREN ZUR HERSTELLUNG VON 3,4-DICHLOR-N-(2-CYANOPHENYL)-5-ISOTHIAZOLCARBOXAMID**
METHOD FOR PRODUCING 3,4-DICHLORO-N-(2-CYANOPHENYL)-5-ISOTHIAZOLECARBOXAMIDE
PROCÉDÉ DE PRÉPARATION DE 3,4-DICHLORO-N-(2-CYANOPHÉNYL)-5-ISOTHIAZOLOCARBOXAMIDE

(30) Priorität: 06.04.2018 EP 18166110
(43) Veröffentlichungstag der Anmeldung: 17.02.2021
(73) Patentinhaber: Bayer Aktiengesellschaft, 51373 Leverkusen (DE); Bayer CropScience Aktiengesellschaft, 40789 Monheim am Rhein (DE)
(72) Erfinder: FARIDA, Taraneh, 50259 Pulheim-Geyen (DE); LITTMANN, Martin, 51375 Leverkusen (DE); SANLI, Ali, 51371 Leverkusen (DE); PABST, Kyra Larissa, 50735 Köln (DE); LUDWIG, Jürgen, 51519 Odenthal (DE); SCHNATTERER, Albert, 51373 Leverkusen (DE)
(74) Vertreter: BIP Patents
(86) Internationale Anmeldenummer: PCT/EP2019/058245
(87) Internationale Veröffentlichungsnummer: WO 2019/192988

(56) Entgegenhaltungen:
- WO-A2-99/24413
- CN-A- 102 942 565

## Beschreibung

Die vorliegende Erfindung betrifft ein neues, einstufiges Verfahren zur Herstellung und Isolierung von 3,4-Dichlor-*N*-(2-cyanophenyl)-5-isothiazolcarboxamid (Isotianil), das mikrobizide und pflanzenstärkende (Host Defense Inducer) Eigenschaften aufweist. Bei dem erfindungsgemäßen Verfahren ist die Abfallmenge des Produktionsprozesses (z.B. Lösungsmittel) deutlich reduziert. Das Produkt wird in einer hohen Ausbeute und Reinheit geliefert (minimale Menge an Nebenprodukten und Verunreinigungen). Gegenüber bestehenden Herstellungsverfahren hat das erfindungsgemäße Verfahren den Vorteil einer geringen Korrosivität sowie einer hohen Ausbeute und einer hohen Selektivität.

Die Synthese von Isotianil wurde in verschiedenen Patentanmeldungen beschrieben. Zum Beispiel ist es bekannt, dass 3,4-Dichlor-*N*-(2-cyanophenyl)-5-isothiazolcarboxamid der Formel (I) durch die Reaktion von 3,4-Dichlorisothiazol-5-carbonylchlorid mit Anthranilamid in der Gegenwart eines Säureakzeptors und eines aprotischen Solvens erhalten werden kann, wobei das aus dieser Reaktion hervorgehende *N*-[2-(aminocarbonyl)phenyl]-3,4-dichlor-5-isothiazolcarboxamid in einem zweiten Schritt noch mit einem Dehydratisierungsmittel umgesetzt werden muss (cf. WO 2004/002968).

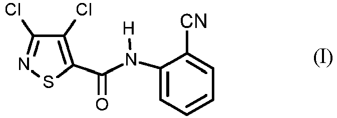

In diesem Verfahren enthält das Filtrat überschüssiges Vilsmeier-Reagenz sowie SO₂ und Salzsäure (HCl), die hoch korrosiv sind. Weiterhin wird eine wässrige Aufarbeitung als unpraktikabel beschrieben, da unter diesen Bedingungen das *N*-Formyl-Nebenprodukt in signifikanten Mengen gebildet wird. Ferner führt die wässrige Aufarbeitung mit notwendiger Neutralisation zu einer erhöhten Abfallmenge.

Während in der Theorie die nicht-wässrige Aufarbeitung der hoch korrosiven Reaktionsmischung und die Isolierung des Produktes möglich ist, ist diese Art der Aufarbeitung jedoch großtechnisch nicht praktikabel und nicht ökonomisch sinnvoll.

CN102942565 beschreibt Herstellungsverfahren von 3,4-Dichlorisothiazol-Derivativen und deren Anwendung als Pestizide, Bakterizide, Mittel gegen Pflanzenviren und Pflanzenaktivatoren. Ferner wird eine Technologie zur Mischung der Verbindung(en) mit landwirtschaftlich verwendbaren Zusatzstoffen oder synergistischen Verbindungen zur Herstellung von Pestiziden, Bakteriziden, Mitteln gegen Pflanzenviren und Pflanzenaktivatoren beschrieben.

Weiterhin ist es bekannt, dass 3,4-Dichlor-*N*-(2-cyanophenyl)-5-isothiazolcarboxamid erhalten werden kann durch die Reaktion von
3,4-Dichlorisothiazol-5-carbonylchlorid der Formel (11) 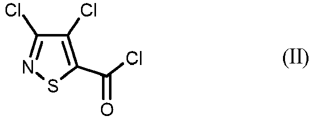
mit 2-Cyanoanilin (2-Aminobenzonitril) der Formel (III) 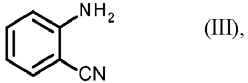
in der Gegenwart eines aprotischen Solvens (cf. WO 99/24413).

Das in WO 99/24413 beschriebene Verfahren hat die Nachteile, dass eine große Menge an Hilfsbase erforderlich ist, eine lange Reaktionszeit benötigt wird, die Reaktionsführung aufwendig ist und ein höherer Anteil an Nebenprodukten entsteht Weiterhin entstehen durch die verdünnte Fahrweise hohe Fertigungskosten und eine vergleichsweise große Abwassermenge.(cf. Example 1 of WO 99/24413).

Dementsprechend bestand immer noch ein Bedarf an einem verbesserten Prozess, der die Herstellung von 3,4-Dichlor-*N*-(2-cyanophenyl)-5-isothiazolcarboxamid in guter Ausbeute mit minimalen Mengen an Nebenprodukten und Verunreinigungen erlaubt, ohne die Verwendung teurer Lösungsmittel oder bei der Aufarbeitung oder Abfallbehandlung schwierig handhabbarer Solventien und Rückstände (z.B. SOCl₂ und SO₂). Gleichzeitig sollte die Abfallmenge pro Kilogramm Produkt gegenüber bekannten Verfahren niedrig oder zumindest nicht erhöht sein.

Bei der Übertragung von Reaktionen in den industriellen Maßstab ist es von besonderer Bedeutung, dass die Reaktionen auch in Stahlreaktoren statt in Glasgefäßen durchgeführt werden können, hierfür ist neben der generellen Reduktion an Abfall eine Kontrolle der korrosiven Eigenschaften sowie eine einfache Reaktionsführung wichtig. Diese Faktoren haben sowohl ökonomische (weniger Kosten) als auch ökologische Effekte (weniger Umweltbelastung).

Weiterhin besteht immer ein Bedarf an Verbesserung der Raum-Zeit-Ausbeute und des Durchsatzes.

Deswegen ist das Ziel der Erfindung, ein Verfahren mit reduzierter Abfallmenge und tolerablen korrosiven Eigenschaften sowie einfacher Reaktionsführung bereitzustellen, insbesondere für den industriellen Maßstab.

Die Berechnung der Abfallmenge wie oben beschrieben, beinhaltet nicht nur die jeweiligen Mengen an Lösungsmitteln, Edukten und Rückständen, sondern auch wässrige und organische Phasen und die benötigen Chemikalien für ihre Verdünnung und/oder Neutralisierung oder Nachbehandlung.

Soweit nicht anders definiert, ist unter Raumtemperatur eine Temperatur von 20°C bis 22°C zu verstehen.

Weiterhin sind in der vorliegenden Beschreibung bevorzugte Bereiche verschiedener Parameter so zu verstehen, dass sie frei kombiniert werden können, unabhängig vom Grad der Bevorzugung. Allerdings ist zumindest die Kombination der am meisten bevorzugten Ausgestaltungen als bevorzugte Ausführungsform des gesamten Prozesses zu verstehen, wie auch Kombinationen von bevorzugten Bereichen gleicher Ebene.

Das erfindungsgemäße Verfahren zeichnet sich durch eine beschleunigte Reaktionsführung, minimalem Einsatz von Lösungsmitteln und Katalysatoren sowie schneller Entfernung korrosiver Reaktionsprodukte aus, sodass ein hoher Durchsatz bei materialschonender Fahrweise ermöglicht wird.

Gemäß der vorliegenden Erfindung beinhaltet das Verfahren für die Herstellung von Isotianil die Schritte
a) Reaktion in einem organischen, aprotischen Lösungsmittel, von
   3,4-Dichlorisothiazol-5-carbonylchlorid der Formel (11) 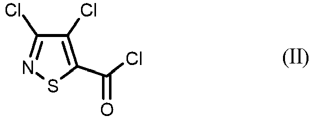
   mit 2-Cyanoanilin (2-Aminobenzonitril) der Formel (III) 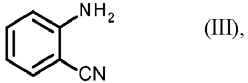
   unter Rückfluss und gegebenenfalls vermindertem Druck
   und,
b) Filtration und Waschen.

In Schritt (a) wird vorzugsweise einer der Reaktanden im Lösungsmittel vorgelegt und erwärmt, und der zweite Reaktand anschließend zudosiert.

### Schritt (a)

Das organische Lösungsmittel ist ausgewählt aus der Gruppe bestehend aus Benzol, Toluol, *o*-Xylol, *m-*Xylol und *p*-Xylol. Noch weiter bevorzugt ist das organische Lösungsmittel ausgewählt aus der Gruppe bestehend aus Toluol, *o*-Xylol, *m*-Xylol und *p*-Xylol. Das am meisten bevorzugte organische Lösungsmittel der Erfindung ist Toluol.

Bei der Durchführung des erfindungsgemäßen Verfahrens können die Temperaturen in einem relativ breiten Bereich variiert werden.

Bei der Durchführung des erfindungsgemäßen Verfahrens sind die Temperaturen in Schritt (a) im Allgemeinen im Bereich zwischen 20°C und 160°C, bevorzugt im Bereich von 40°C bis 120°C, weiter bevorzugt im Bereich von 50°C bis 115°C, and am meisten bevorzugt im Bereich von 60°C bis 95°C. In einer besonders bevorzugten Ausgestaltung wird Schritt (a) in einem Bereich von 70°C bis 90°C durchgeführt.

Bei Reaktionstemperaturen unter dem Siedepunkt des Lösemittels wird der Druck an die verminderte Temperatur so angepasst, dass das Reaktionsgemisch siedet. Dabei wird bevorzugt die Siedepunktserhöhung durch gelöste Reaktanden, Reagenzien und Produkte berücksichtigt. Beim Arbeiten unter vermindertem Druck kann die Reaktionstemperatur verringert werden und dadurch ein positiver Beitrag zur Energiebilanz erreicht werden. Weiterhin kann durch das Entfernen flüchtiger Reaktionsprodukte das Reaktionsgleichgewicht verschoben werden. Dadurch verringert sich die Reaktionszeit und die Raum-Zeit-Ausbeute wird verbessert.

In einer bevorzugten Ausführungsform wird Schritt (a) bei 10 mbar bis 700 mbar durchgeführt, weiter bevorzugt bei 150 mbar bis 650 mbar, noch weiter bevorzugt bei 150 mbar bis 500 mbar, und am meisten bevorzugt bei 200 mbar bis 450 mbar, insbesondere, wenn Toluol verwendet wird, wobei der Bereich, wie auch im folgenden Text, den angelegten Druck angibt und nicht die Reduktion des Drucks ausgehend vom Normaldruck. Wenn die Reaktion im Bereich von 200 mbar bis 450 mbar durchgeführt wird, ist die Reaktionstemperatur bevorzugt zwischen 70 °C und 90 °C, bevorzugt mit Toluol als Lösungsmittel.

Allerdings wird der Fachmann abhängig vom absoluten Druck und dem im Verfahren verwendeten Lösungsmittel die Temperatur und den Druck so anpassen, dass die gewünschte Siedetemperatur erreicht wird. Um dennoch akzeptable Reaktionszeiten sowie sichere Reaktionsbedingungen zu erreichen (z.B. nicht zu viele Nebenprodukte durch thermische Zersetzung), ist die Reaktionstemperatur bevorzugt zwischen 40°C und 160°C, während aus ökonomischen Gründen (Energieverbrauch, Rückgewinnung des Lösungsmittels etc.) der verminderte Druck normalerweise in einem Bereich von 150 mbar bis 500 mbar liegt, weiter bevorzugt ist die Temperatur zwischen 70 °C und 90 °C und der Druck zwischen 200 mbar und 450 mbar.

Die Vorteile der Reaktionsführung unter Rückfluss sind ein geringer Anteil an Nebenprodukten, eine schnellere Reaktion und die (Rück)gewinnung / Abführung der ausgegasten Salzsäure.

Weiterhin gelingt es bei diesem Verfahren, nur einen geringen Überschuss oder sogar einen Unterschuss des Säurechlorids einzusetzen und so eine nahezu ideale Stöchiometrie zu realisieren.

Das Verhältnis von Aminobenzonitril zu 3,4-Dichlorisothiazol-5-carbonylchlorid liegt vorzugsweise zwischen 1:0,80 und 1:1,20, weiter bevorzugt zwischen 1:0,90 und 1:1,10, und besonders bevorzugt zwischen 1:0,95 und 1:1,04.

Die Menge an Lösungsmittel in kg im Verhältnis zu den Reaktanden in kmol liegt vorzugsweise zwischen 1500:1 bis 100:1, weiter bevorzugt zwischen 1000:1 und 150:1, und besonders bevorzugt zwischen 826:1 bis 238:1.

Die Reaktionszeit (Zudosierzeit plus Nachrührzeit) beträgt vorzugsweise zwischen 12 h und 2 h, weiter bevorzugt zwischen 8 h und 2 h, und besonders bevorzugt 6 h.

### Schritt (b)

Nach Beendigung der Reaktion und Abkühlen auf Raumtemperatur, wird das aus dem Reaktionsgemisch ausgefallene Produkt filtriert und der Filterkuchen gewaschen. Bevorzugt wird der Filterkuchen mit einem organischen Lösungsmittel gewaschen, vorzugsweise mit Toluol, Methanol, Ethanol, *iso-* oder *n*-Propanol, besonders bevorzugt mit Toluol. Die Filtration erfolgt vorzugsweise durch Abnutschen.

Die Vorteile des Schrittes b) des erfindungsgemäßen Verfahrens sind, dass nicht zwingend ein zweites Lösungsmittel verwendet werden muss, und daher kein Lösungsmittelgemisch entsteht, welches man im Anschluss wieder trennen müsste. Die Rückgewinnung des Lösungsmittels ist damit deutlich erleichtert.

Das erfindungsgemäße Verfahren ist durch eine Anzahl von Vorteilen gekennzeichnet. Es ermöglicht die Herstellung von 3,4-Dichlor-*N*-(2-cyanophenyl)-5-isothiazolcarboxamid (Isotianil) in einer sehr guten Ausbeute und hoher Reinheit, während die Kosten durch günstigere Lösungsmittel, geringeren Lösungsmittelanteil, weniger Reagenzien, einfachere Aufarbeitung und weniger Abfall pro Kilogramm Produkt reduziert werden, wobei damit auch ein ökologischer Effekt einhergeht.

Ohne Probleme kann das erfindungsgemäße Verfahren im industriellen Maßstab durchgeführt werden, neben anderen Gründen durch die reduzierte Korrosivität.

Der Raum-Zeit-Durchsatz verbessert sich von 20 g/l auf 182 g/l in der gleichen Zeit.

Die Abfallmenge verringert sich von 27,5 kg auf 0,2 kg bis 1,6 kg pro Kilogramm Isotianil.

Die Ausbeute erhöht sich von 89% auf 98%, bei einer Reinheit von 99.8% .

In einer bevorzugten Ausgestaltung der vorliegenden Erfindung beinhaltet das Verfahren für die Herstellung von Isotianil die folgenden Schritte:
a) Reaktion in einem organischen, aprotischen Lösungsmittel, von
   (a) 3,4-Dichlorisothiazol-5-carbonylchlorid der Formel (II) 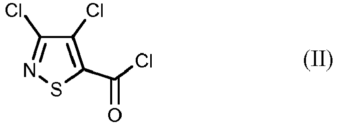 mit 2-Cyanoanilin (2-Aminobenzonitril) der Formel (III) 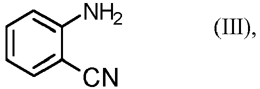 unter Rückfluss und gegebenenfalls vermindertem Druck
   und,
b) Filtration und Waschen (b),

wobei das organische, aprotische Lösungsmittel ausgewählt ist aus einer Gruppe bestehend aus Benzol, Toluol, *o*-Xylol, *m*-Xylol und *p*-Xylol, vorzugsweise Toluol, und
Schritt (a) durchgeführt wird in einem Bereich von 40 °C bis 120 °C und bei 150 mbar bis 650 mbar, und in Schritt (b) die Temperatur auf 20°C bis 22°C eingestellt wird und das ausgefallene Produkt abfiltriert wird, bevorzugt mit einer Nutsche, und der Filterkuchen gewaschen mit Toluol, Methanol, Ethanol, *iso-* oder *n*-Propanol, besonders bevorzugt mit Toluol.

In einer besonders bevorzugten Ausführungsform der vorliegenden Erfindung beinhaltet das Verfahren für die Herstellung von Isotianil die folgenden Schritte:
a) Reaktion in einem organischen, aprotischen Lösungsmittel, von
   (a) 3,4-Dichlorisothiazol-5-carbonylchlorid der Formel (II) 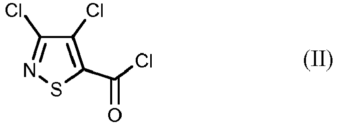 mit 2-Cyanoanilin (2-Aminobenzonitril) der Formel (III) 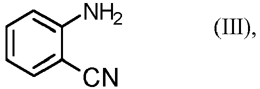 unter Rückfluss und gegebenenfalls vermindertem Druck
   und,
b) Filtration und Waschen (b),

wobei das organische, aprotische Lösungsmittel Toluol ist, und
Schritt (a) durchgeführt wird in einem Bereich von 70 °C bis 90 °C und bei 200 mbar bis 450 mbar, und in Schritt (b) die Temperatur auf 20°C bis 22°C eingestellt wird und das ausgefallene Produkt abfiltriert wird, bevorzugt mit einer Nutsche, und der Filterkuchen gewaschen mit Toluol,
und das Verhältnis von Aminobenzonitril zu 3,4-Dichlorisothiazol-5-carbonylchlorid zwischen 1:0,90 und 1:1,10 liegt, und
die Menge an Lösungsmittel in kg im Verhältnis zu den Reaktanden in kmol zwischen 1000:1 und 150:1 liegt, und
die Reaktionszeit (Zudosierzeit plus Nachrührzeit) zwischen 8 h und 2 h beträgt.

In den oben genannten Ausführungsformen wird in der Reaktionsführung vorzugsweise ein Reaktand vorgelegt und der zweite mittels einer getauchten Zuleitung (Tauchung) in das Reaktionsgemisch dosiert, wobei Aminobenzonitril als Schmelze vorgelegt oder auch dosiert werden kann. Bevorzugt wird das Chlorid vorgelegt.

In der besten Ausführungsform der vorliegenden Erfindung beinhaltet das Verfahren für die Herstellung von Isotianil die demnach folgenden Schritte:
a) Reaktion in einem organischen, aprotischen Lösungsmittel, von
   (a) 3,4-Dichlorisothiazol-5-carbonylchlorid der Formel (II) 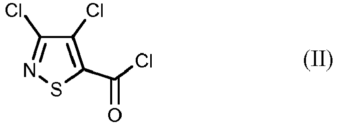 mit 2-Cyanoanilin (2-Aminobenzonitril) der Formel (III) 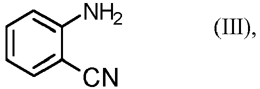 unter Rückfluss und gegebenenfalls vermindertem Druck, wobei das Aminobenzonitril in Schmelze oder in Lösung, bevorzugt in Lösung, mittels Tauchung zugegeben wird.
   und,
b) Filtration und Waschen (b),

wobei das organische, aprotische Lösungsmittel ausgewählt Toluol ist, und
Schritt (a) durchgeführt wird in einem Bereich von 70 °C bis 90 °C und bei 200 mbar bis 450 mbar, und das Verhältnis von Aminobenzonitril zu 3,4-Dichlorisothiazol-5-carbonylchlorid zwischen 1:0,95 und 1:1,04 liegt, und
die Menge an Lösungsmittel in kg im Verhältnis zu den Reaktanden in kmol zwischen 826:1 und 238:1 liegt, und
die Reaktionszeit (Zudosierzeit plus Nachrührzeit) 6 h beträgt.
in Schritt (b) die Temperatur auf 20°C bis 22°C eingestellt wird und das ausgefallene Produkt abfiltriert wird, bevorzugt mit einer Nutsche, und der Filterkuchen gewaschen mit Toluol.

Die im Folgenden beschriebenen Beispiele illustrieren die vorliegende Erfindung detaillierter ohne sie dabei einzuschränken.

### Synthese- und Vergleichsbeispiele

### Beispiel 1 (erfindungsgemäß): Vorlage Aminobenzonitril in Toluol:

59,2 g (0,5 mol, 99,8 %ig) Aminobenzonitril in 552,7 g (5,99 mol, 99,8 %ig) Toluol vorgelegt. Es werden innerhalb von 4 h bei 80 °C Innentemperatur (Badtemperatur 100-114 °C) und 345 mbar (starker Rückfluss) 114,4 g (0,48 mol, 90 %ig in Toluol) DCIT-Chlorid zugetropft. Anschließend 2 h unter Rückfluss bei 80 °C und 345 mbar nachgerührt. Die Reaktionsmischung wurde auf Raumtemperatur abgekühlt und filtriert. Der Nutschkuchen wurde 2-mal mit Methanol (Verdrängungswäsche) gewaschen.
Ausbeute: 98,8 % d. Th.
Reinheit: 99,9 %
Abfallmenge: 1,6 kg / kg Isotianil

### Beispiel 2 (erfindungsgemäß): Vorlage DCIT-Chlorid in Toluol und Dosierung Aminobenzonitril in Toluol:

290,0 g (1,28 mol, 95,1 %ig in Toluol) DCIT-Chlorid und 51,9 g (0,56 mol, 100 %ig) Toluol wurden vorgelegt. 145,3 g (1,23 mol, 100 %ig) Aminobenzonitril in 530,7 g (0,56 mol, 100 %ig) in Toluol werden innerhalb von 4 h bei 80 °C Innentemperatur (Badtemperatur 100-114 °C) und 345 mbar (starker Rückfluss) getaucht zugetropft. Anschließend 2 h unter Rückfluss bei 80 °C und 345 mbar nachgerührt. Die Reaktionsmischung wurde auf Raumtemperatur abgekühlt und filtriert. Der Nutschkuchen wurde 2-mal mit Toluol (Verdrängungswäsche) gewaschen.
Ausbeute isoliert: 98,5 % d. Th. (1,5 % d. Th. In der Mutterlauge und Waschfiltrat)
Reinheit: 99,8 %
Abfallmenge: 0,2 kg / kg Isotianil

### Beispiel 3 (erfindungsgemäß): Vorlage DCIT-Chlorid in Toluol und Dosierung Aminobenzonitril als Schmelze:

292,9 g (1,29 mol, 95,1 %ig in Toluol) DCIT-Chlorid und 475,8 g (5,16 mol, 100 %ig) Toluol wurden bei RT vorgelegt. 146,8 g (1,24 mol, 100 %ig) Aminobenzonitril als Schmelze werden innerhalb von 4 h bei 80 °C Innentemperatur (Badtemperatur 100-114 °C) und 350 mbar (starker Rückfluss) getaucht zugetropft. Anschließend 2 h unter Rückfluss bei 80 °C und 345 mbar nachgerührt. Die Reaktionsmischung wurde auf Raumtemperatur abgekühlt und filtriert. Der Nutschkuchen wurde 2-mal mit Toluol (Verdrängungswäsche) gewaschen.
Ausbeute isoliert: 95,6 % d. Th. (3,22 % d. Th. In der Mutterlauge und Waschfiltrat)
Reinheit: 99,3 %
Abfallmenge: 0,2 kg / kg Isotianil

### Beispiel 4 (nicht erfindungsgemäß):

Vorlage Aminobenzonitril in Methylacetat als LM 59,2 g (0,5 mol, 99,8 %ig) Aminobenzonitril in 444,3 g (5,99 mol, 99,8 %ig) Methylacetat vorgelegt. Es werden innerhalb von 4 h bei 45-47 °C Innentemperatur (Badtemperatur 88-85 °C) und 600-605 mbar (starker Rückfluss) 126,8 g (0,48 mol, 81,2 %ig in Methylacetat) DCIT-Chlorid zugetropft. Anschließend 2 h unter Rückfluss bei 45-47 °C und 600-605 mbar nachgerührt. Die Reaktionsmischung wurde auf Raumtemperatur abgekühlt und filtriert. Der Nutschkuchen wurde 2-mal mit Methanol (Verdrängungswäsche) gewaschen.
Ausbeute: 94,2 % d. Th. (1,22 % d. Th. In der Mutterlauge und Waschfiltrat)
Reinheit: 100 %
Abfallmenge: 0,3 kg / kg Isotianil

### Beispiel 5 (nicht erfindungsgemäß):

Vorlage Aminobenzonitril in Chlorbenzol als LM 59,19 g (0,5 mol, 99,8 %ig) Aminobenzonitril in 675,14 g (5,99 mol, 99,8 %ig) Chlorbenzol vorgelegt. Es werden innerhalb von 4 h bei 79-80 °C Innentemperatur (Badtemperatur 101-102 °C) und 155-165 mbar (starker Rückfluss) 126,8 g (0,48 mol, 81,2 %ig in Chlorbenzol) DCIT-Chlorid zugetropft. Anschließend 2 h unter Rückfluss bei 79-80 °C und 155-170 mbar nachgerührt. Die Reaktionsmischung wurde auf Raumtemperatur abgekühlt und filtriert. Der Nutschkuchen wurde 2-mal mit Methanol (Verdrängungswäsche) gewaschen.
Ausbeute: 92,0 % d. Th. (4,6 % d. Th. In der Mutterlauge und Waschfiltrat)
Reinheit: 100 %
Abfallmenge: 0,4 kg / kg Isotianil

### Beispiel 6 (nicht erfindungsgemäß):

Vorlage Aminobenzonitril in Di-n-butylether als LM 47,4 g (0,4 mol, 99,8 %ig) Aminobenzonitril in 624,9 g (4,79 mol, 99,8 %ig) Di-n-butylether vorgelegt. Es werden innerhalb von 4 h bei 79-80 °C Innentemperatur (Badtemperatur 101-102 °C) und 105 mbar (starker Rückfluss) 101,5 g (0,38 mol, 81,2 %ig in Di-n-butylether) DCIT-Chlorid zugetropft. Anschließend 2 h unter Rückfluss bei 79-80 °C und 105 mbar nachgerührt. Die Reaktionsmischung wurde auf Raumtemperatur abgekühlt und filtriert. Der Nutschkuchen wurde 2-mal mit Methanol (Verdrängungswäsche) gewaschen.
Ausbeute: 95,4 % d. Th. (1,5 % d. Th. in der Mutterlauge und Waschfiltrat)
Reinheit: 99,4 %
Abfallmenge: 0,4 kg / kg Isotianil

### Beispiel 7 (erfindungsgemäß): Getauchte Zugabe von 2-Aminobenzonitril als toluolische Lösung

290 g (1,27 mol, 95,1 %ig) DCIT-Chlorid und 46 g (0,5 mol, 100 %ig) Toluol werden bei Raumtemperatur vorgelegt und auf 80 °C aufgeheizt. Eine Lösung aus 530,55 g (5,76 mol, 100 %ig) Toluol und 145,34 g (1,23 mol, 99,8 %ig) Aminobenzonitril werden innerhalb von 4 h bei 79-80 °C Innentemperatur (Badtemperatur 101-102 °C) und 105 mbar (starker Rückfluss) über eine Tauchung zudosiert. Anschließend 2 h unter Rückfluss bei 79-80 °C und 100-110 mbar nachgerührt. Die Suspension wird auf 20 °C abgekühlt, filtriert und zweimal mit Toluol gewaschen.
Ausbeute: 97,5 % d. Th. (1,50 % d. Th. in der Mutterlauge und Waschfiltrat)
Reinheit: 99,8 %
Abfallmenge: 0,2 kg / kg Isotianil

### Versleichsbeispiel

### (Aminobenzonitril-Verfahren)

In ein Gemisch aus 20,8 g (0,1725 Mol) 2-Cyanoanilin und 250 ml Pyridin werden bei 5 bis 10 °C unter Rühren 38,1 g (0,15 Mol) 3,4-Dichlor-isothiazol-5-carbonsäurechlorid innerhalb von 10 Minuten eingetropft. Nach beendeter Zugabe versetzt man das Reaktionsgemisch mit 70 ml absolutem Tetrahydrofuran und 30 ml Pyridin, lässt auf Raumtemperatur erwärmen und rührt dann 75 Minuten bei Raumtemperatur. Anschließend wird das Reaktionsgemisch unter vermindertem Druck eingeengt. Der verbleibende Rückstand wird mit 800 ml Wasser und 800 ml Essigsäureethylester verrührt. Der in dem Zweiphasen-Gemisch enthaltene Niederschlag wird abfiltriert, mit Essigsäureethylester gewaschen und getrocknet. Man erhält auf diese Weise 31,7 g eines kristallinen Produktes vom Schmelzpunkt 191 bis 193 °C.

Aus dem zweiphasigen Filtrat wird die wässrige Phase abgetrennt und zweimal mit je 150 ml Essigsäureethylester extrahiert. Die vereinigten organischen Phasen werden über Natriumsulfat getrocknet und dann unter vermindertem Druck eingeengt. Der verbleibende Rückstand wird mit 100 ml Petrolether und 25 ml Essigsäureethylester nachgewaschen und getrocknet.

Man erhält auf diese Weise insgesamt 40 g (89 % der Theorie) an 3,4-Dichlor-isothiazol-5-carbonsäure-(2-cyano-anilid) in Form einer Festsubstanz vom Schmelzpunkt 191 bis 193 °C.
Ausbeute isoliert: 89 % d. Th.
Abfallmenge: 27,5 kg / kg Isotianil

## Patentansprüche

1. Verfahren für die Herstellung von Isotianil, das die Schritte
(a) Reaktion in einem organischen, aprotischen Lösungsmittel, von
3,4-Dichlorisothiazol-5-carbonylchlorid der Formel (II) 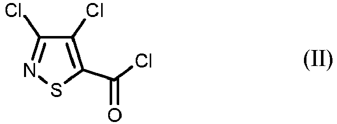
mit 2-Cyanoanilin (2-Aminobenzonitril) der Formel (III) 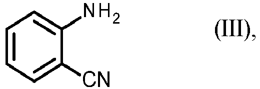
unter Rückfluss und gegebenenfalls vermindertem Druck
und
(b) Filtration und Waschen beinhaltet,
wobei in Schritt (a) vorzugsweise einer der Reaktanden im Lösungsmittel vorgelegt und erwärmt wird, und der zweite Reaktand anschließend zudosiert wird, und **dadurch gekennzeichnet, dass** das organische, aprotische Lösungsmittel ausgewählt ist aus einer Gruppe bestehend aus Benzol, Toluol, *o*-Xylol, *m*-Xylol und *p*-Xylol, vorzugsweise Toluol.

2. Verfahren nach Anspruch 1, **dadurch gekennzeichnet, dass** Schritt (a) durchgeführt wird in einem Bereich von 40 °C bis 120 °C und bei 150 mbar bis 650 mbar.

3. Verfahren nach einem der Ansprüche 1 oder 2, **dadurch gekennzeichnet, dass** in Schritt (b) die Temperatur auf 20°C bis 22°C eingestellt wird und das ausgefallene Produkt abfiltriert wird, bevorzugt mit einer Nutsche, und der Filterkuchen mit Toluol, Methanol, Ethanol, *iso-* oder *n-*Propanol, besonders bevorzugt mit Toluol, gewaschen wird.

4. Verfahren nach einem der Ansprüche 1 bis 3, **dadurch gekennzeichnet, dass** ein Reagenz in Schmelze oder in Lösung mittels Tauchung zugegeben wird.

5. Verfahren nach Anspruch 4, **dadurch gekennzeichnet, dass** das Aminobenzonitril in Schmelze oder in Lösung mittels Tauchung zugegeben wird

6. Verfahren nach Anspruch 1, **dadurch gekennzeichnet, dass**
Schritt (a) durchgeführt wird in einem Bereich von 40 °C bis 120 °C und bei 150 mbar bis 650 mbar, und
in Schritt (b) die Temperatur auf 20°C bis 22°C eingestellt wird und das ausgefallene Produkt abfiltriert wird, bevorzugt mit einer Nutsche, und der Filterkuchen mit Toluol, Methanol, Ethanol, *iso-* oder *n*-Propanol, besonders bevorzugt mit Toluol, gewaschen wird.

7. Verfahren nach einem der Ansprüche 1 oder 6, **dadurch gekennzeichnet, dass** das organische, aprotische Lösungsmittel Toluol ist, und
Schritt (a) durchgeführt wird in einem Bereich von 70 °C bis 90 °C und bei 200 mbar bis 450 mbar, und
in Schritt (b) die Temperatur auf 20°C bis 22°C eingestellt wird und das ausgefallene Produkt abfiltriert wird, bevorzugt mit einer Nutsche, und der Filterkuchen mit Toluol gewaschen wird,
und das Verhältnis von Aminobenzonitril zu 3,4-Dichlorisothiazol-5-carbonylchlorid zwischen 1:0,90 und 1: 1,10 liegt, und
die Menge an Lösungsmittel in kg im Verhältnis zu den Reaktanden in kmol zwischen 1000: 1 und 150:1 liegt, und
die Reaktionszeit (Zudosierzeit plus Nachrührzeit) zwischen 8 h und 2 h beträgt.

8. Verfahren nach einem der Ansprüche 1, 6 oder 7, **dadurch gekennzeichnet, dass** ein Reagenz in Schmelze oder in Lösung mittels Tauchung zugegeben wird,
und das organische, aprotische Lösungsmittel Toluol ist, und
Schritt (a) durchgeführt wird in einem Bereich von 70 °C bis 90 °C und bei 200 mbar bis 450 mbar, und
das Verhältnis von Aminobenzonitril zu 3,4-Dichlorisothiazol-5-carbonylchlorid zwischen 1:0,95 und 1:1,04 liegt, und
die Menge an Lösungsmittel in kg im Verhältnis zu den Reaktanden in kmol zwischen 826:1 und 238:1 liegt, und
die Reaktionszeit (Zudosierzeit plus Nachrührzeit) 6 h beträgt, und
in Schritt (b) die Temperatur auf 20°C bis 22°C eingestellt wird und das ausgefallene Produkt abfiltriert wird, bevorzugt mit einer Nutsche, und der Filterkuchen mit Toluol gewaschen wird.

9. Verfahren nach Anspruch 8, **dadurch gekennzeichnet, dass** das Aminobenzonitril in Schmelze oder in Lösung mittels Tauchung zugegeben wird,
und das organische, aprotische Lösungsmittel Toluol ist, und
Schritt (a) durchgeführt wird in einem Bereich von 70 °C bis 90 °C und bei 200 mbar bis 450 mbar, und
das Verhältnis von Aminobenzonitril zu 3,4-Dichlorisothiazol-5-carbonylchlorid zwischen 1:0,95 und 1:1,04 liegt, und
die Menge an Lösungsmittel in kg im Verhältnis zu den Reaktanden in kmol zwischen 826:1 und 238:1 liegt, und
die Reaktionszeit (Zudosierzeit plus Nachrührzeit) 6 h beträgt, und
in Schritt (b) die Temperatur auf 20°C bis 22°C eingestellt wird und das ausgefallene Produkt abfiltriert wird, bevorzugt mit einer Nutsche, und der Filterkuchen mit Toluol gewaschen wird.

10. Verfahren nach Anspruch 8, **dadurch gekennzeichnet, dass** das Aminobenzonitril in Lösung mittels Tauchung zugegeben wird,
und das organische, aprotische Lösungsmittel Toluol ist, und
Schritt (a) durchgeführt wird in einem Bereich von 70 °C bis 90 °C und bei 200 mbar bis 450 mbar, und
das Verhältnis von Aminobenzonitril zu 3,4-Dichlorisothiazol-5-carbonylchlorid zwischen 1:0,95 und 1:1,04 liegt, und
die Menge an Lösungsmittel in kg im Verhältnis zu den Reaktanden in kmol zwischen 826:1 und 238:1 liegt, und
die Reaktionszeit (Zudosierzeit plus Nachrührzeit) 6 h beträgt, und
in Schritt (b) die Temperatur auf 20°C bis 22°C eingestellt wird und das ausgefallene Produkt abfiltriert wird, bevorzugt mit einer Nutsche, und der Filterkuchen mit Toluol gewaschen wird.

## Claims

1. Method for preparing isotianil which includes the steps of
(a) reaction in an organic aprotic solvent, of 3,4-dichloroisothiazole-5-carbonyl chloride of the formula (II) 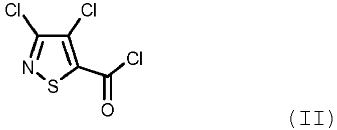 with 2-cyanoaniline (2-aminobenzonitrile) of the formula (III) 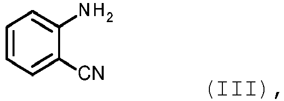 under reflux and optionally reduced pressure,
and
(b) filtration and washing,
wherein in step (a) preferably one of the reactants is initially charged in the solvent and heated, and the second reactant is subsequently metered in, **characterized in that** the organic aprotic solvent is selected from a group consisting of benzene, toluene, *o*-xylene, *m*-xylene and *p*-xylene, preferably toluene.

2. Method according to Claim 1, **characterized in that** step (a) is conducted in a range from 40°C to 120°C and at 150 mbar to 650 mbar.

3. Method according to either of Claims 1 and 2, **characterized in that** in step (b) the temperature is adjusted to 20°C to 22°C and the precipitated product is filtered off, preferably using a Nutsche filter, and the filter cake is washed with toluene, methanol, ethanol, isopropanol or n-propanol, particularly preferably with toluene.

4. Method according to any of Claims 1 to 3, **characterized in that** a reagent is added in the form of a melt or in solution by means of immersion.

5. Method according to Claim 4, **characterized in that** aminobenzonitrile is added in the form of a melt or in solution by means of immersion.

6. Method according to Claim 1, **characterized in that** step (a) is carried out in a range from 40°C to 120°C and at 150 mbar to 650 mbar, and
in step (b) the temperature is adjusted to 20°C to 22°C and the precipitated product is filtered off, preferably using a Nutsche filter, and the filter cake is washed with toluene, methanol, ethanol, *iso*propanol or *n*-propanol, particularly preferably with toluene.

7. Method according to either of Claims 1 and 6, **characterized in that** the organic aprotic solvent is toluene, and
step (a) is carried out in a range from 70°C to 90°C and at 200 mbar to 450 mbar, and
in step (b) the temperature is adjusted to 20°C to 22°C, and the precipitated product is filtered off, preferably using a Nutsche filter, and the filter cake is washed with toluene,
and the ratio of aminobenzonitrile to 3,4-dichloroisothiazole-5-carbonyl chloride is between 1:0.90 and 1:1.10, and
the amount of solvent in kg in the ratio to the reactants in kmol is between 1000:1 and 150:1, and
the reaction time (metered addition time plus further stirring time) is between 8 h and 2 h.

8. Method according to any of Claims 1, 6 or 7, **characterized in that** a reagent is added in the form of a melt or in solution by means of immersion,
and the organic aprotic solvent is toluene, and
step (a) is carried out in a range from 70°C to 90°C and at 200 mbar to 450 mbar, and
the ratio of aminobenzonitrile to 3,4-dichloroisothiazole-5-carbonyl chloride is between 1:0.95 and 1:1.04, and
the amount of solvent in kg in the ratio to the reactants in kmol is between 826:1 and 238:1, and
the reaction time (metered addition time plus further stirring time) is 6 h, and
in step (b) the temperature is adjusted to 20°C to 22°C, and the precipitated product is filtered off, preferably using a Nutsche filter, and the filter cake is washed with toluene.

9. Method according to Claim 8, **characterized in that** aminobenzonitrile is added in the form of a melt or in solution by means of immersion,
and the organic aprotic solvent is toluene, and
step (a) is carried out in a range from 70°C to 90°C and at 200 mbar to 450 mbar, and
the ratio of aminobenzonitrile to 3,4-dichloroisothiazole-5-carbonyl chloride is between 1:0.95 and 1:1.04, and
the amount of solvent in kg in the ratio to the reactants in kmol is between 826:1 and 238:1, and
the reaction time (metered addition time plus further stirring time) is 6 h, and
in step (b) the temperature is adjusted to 20°C to 22°C, and the precipitated product is filtered off, preferably using a Nutsche filter, and the filter cake is washed with toluene.

10. Method according to Claim 8, **characterized in that** aminobenzonitrile is added in solution by means of immersion,
and the organic aprotic solvent is toluene, and
step (a) is carried out in a range from 70°C to 90°C and at 200 mbar to 450 mbar, and
the ratio of aminobenzonitrile to 3,4-dichloroisothiazole-5-carbonyl chloride is between 1:0.95 and 1:1.04, and
the amount of solvent in kg in the ratio to the reactants in kmol is between 826:1 and 238:1, and
the reaction time (metered addition time plus further stirring time) is 6 h, and
in step (b) the temperature is adjusted to 20°C to 22°C, and the precipitated product is filtered off, preferably using a Nutsche filter, and the filter cake is washed with toluene.

## Revendications

1. Procédé de préparation d'isotianil, qui comprend les étapes suivantes :
(a) réaction, dans un solvant aprotique organique, de chlorure de 3,4-dichloroisothiazole-5-carbonyle de Formule (II) 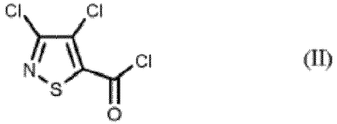 avec de la 2-cyanoaniline (2-aminobenzonitrile) de Formule (III) 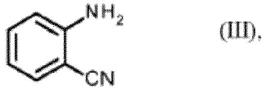 au reflux et éventuellement sous pression réduite,
et
(b) filtration et lavage,
dans lequel, dans l'étape (a), de préférence l'un des réactifs est mis en place dans le solvant et est chauffé, et l'autre réactif est ensuite ajouté, et **caractérisé en ce que** le solvant aprotique organique est choisi dans un groupe consistant en le benzène, le toluène, le *o*-xylène, le *m*-xylène et le *p*-xylène, de préférence le toluène.

2. Procédé selon la revendication 1, **caractérisé en ce que** l'étape (a) est mise en œuvre dans la plage de 40 °C à 120 °C et sous 150 mbar à 650 mbar.

3. Procédé selon l'une des revendications 1 ou 2, **caractérisé en ce que** dans l'étape (b), la température est ajustée à 20 °C à 22 °C et le produit qui a précipité est isolé par filtration, de préférence avec un entonnoir-filtre, et le gâteau de filtration est lavé avec du toluène, du méthanol, de l'éthanol, de l'*iso-* ou du *n*-propanol, d'une manière particulièrement préférée avec du toluène.

4. Procédé selon l'une des revendications 1 à 3, **caractérisé en ce qu'**un réactif est ajouté à l'état fondu ou en solution par immersion.

5. Procédé selon la revendication 4, **caractérisé en ce que** l'aminobenzonitrile est ajouté à l'état fondu ou en solution par immersion.

6. Procédé selon la revendication 1, **caractérisé en ce que**
l'étape (a) est mise en œuvre dans la plage de 40 °C à 120 °C et sous 150 mbar à 650 mbar, et
dans l'étape (b), la température est ajustée à 20 °C à 22 °C et le produit qui a précipité est isolé par filtration, de préférence avec un entonnoir-filtre, et le gâteau de filtration est lavé avec du toluène, du méthanol, de l'éthanol, de l'*iso-* ou du *n*-propanol, d'une manière particulièrement préférée avec du toluène.

7. Procédé selon l'une des revendications 1 ou 6, **caractérisé en ce que** le solvant aprotique organique est le toluène, et
l'étape (a) est mise en œuvre dans la plage de 70 °C à 90 °C et sous 200 mbar à 450 mbar, et
dans l'étape (b), la température est ajustée à 20 °C à 22 °C et le produit qui a précipité est isolé par filtration, de préférence avec un entonnoir-filtre, et le gâteau de filtration est lavé avec du toluène,
et le rapport de l'aminobenzonitrile au chlorure de 3,4-dichloroisothiazole-5-carbonyle est compris entre 1:0,90 et 1:1,10, et
la quantité de solvant par kg, rapportée aux réactifs en kmol, est comprise entre 1000:1 et 150:1, et
le temps de réaction (temps d'addition plus temps de reprise d'agitation) est compris entre 2 heures et 8 heures.

8. Procédé selon l'une des revendications 1, 6 ou 7, **caractérisé en ce qu'**un réactif est ajouté à l'état fondu ou en solution par immersion,
et le solvant aprotique organique est le toluène, et
l'étape (a) est mise en œuvre dans la plage de 70 °C à 90 °C et sous 200 mbar à 450 mbar, et
le rapport de l'aminobenzonitrile au chlorure de 3,4-dichloroisothiazole-5-carbonyle est compris entre 1:0,95 et 1:1,04, et
la quantité de solvant en kg, rapportée aux réactifs en kmol, est comprise entre 826:1 et 238:1, et
le temps de réaction (temps d'addition plus temps de reprise d'agitation) est de 6 heures, et
dans l'étape (b), la température est ajustée à 20 °C à 22 °C et le produit qui a précipité est isolé par filtration, de préférence avec un entonnoir-filtre, et le gâteau de filtration est lavé avec du toluène.

9. Procédé selon la revendication 8, **caractérisé en ce que** l'aminobenzonitrile est ajouté à l'état fondu ou en solution par immersion,
et le solvant aprotique organique est le toluène, et
l'étape (a) est mise en œuvre dans la plage de 70 °C à 90 °C et sous 200 mbar à 450 mbar, et
le rapport de l'aminobenzonitrile au chlorure de 3,4-dichloroisothiazole-5-carbonyle est compris entre 1:0,95 et 1:1,04, et
la quantité de solvant en kg, rapportée aux réactifs en kmol, est comprise entre 826:1 et 238:1, et
le temps de réaction (temps d'addition plus temps de reprise d'agitation) est de 6 heures, et
dans l'étape (b), la température est ajustée à 20 °C à 22 °C et le produit qui a précipité est isolé par filtration, de préférence avec un entonnoir-filtre, et le gâteau de filtration est lavé avec du toluène.

10. Procédé selon la revendication 8, **caractérisé en ce que** l'aminobenzonitrile est ajouté en solution par immersion,
et le solvant aprotique organique est le toluène, et
l'étape (a) est mise en œuvre dans la plage de 70 °C à 90 °C et sous 200 mbar à 450 mbar, et
le rapport de l'aminobenzonitrile au chlorure de 3,4-dichloroisothiazole-5-carbonyle est compris entre 1:0,95 et 1:1,04, et
la quantité de solvant en kg, rapportée aux réactifs en kmol, est comprise entre 826:1 et 238:1, et
le temps de réaction (temps de dosage plus temps de reprise d'agitation) est de 6 heures, et
dans l'étape (b), la température est ajustée à 20 °C à 22 °C et le produit qui a précipité est isolé par filtration, de préférence avec un entonnoir-filtre, et le gâteau de filtration est lavé avec du toluène.
